# EUROPEAN PATENT SPECIFICATION

(11) **EP 4 473 283 B1**
(45) Date of publication and mention of the grant of the patent: **10.12.2025**
(21) Application number: 23712319.5
(22) Date of filing: 31.01.2023
(51) Int. Cl.: G01N 1/31, B01L 9/00, G01N 35/00, G01N 35/04

(54) **A SLIDE COVER AND AN INCUBATION CHAMBER CONTAINING SUCH A SLIDE COVER**
SCHIEBEDECKEL UND INKUBATIONSKAMMER MIT SOLCH EINEM SCHIEBEDECKEL
COUVERCLE DE LAME PORTE-OBJET ET CHAMBRE D'INCUBATION CONTENANT UN TEL COUVERCLE DE LAME PORTE-OBJET

(30) Priority: 04.02.2022 HU 2200032
(43) Date of publication of application: 11.12.2024
(73) Proprietor: 3DHistech Kft., 1141 Budapest (HU)
(72) Inventor: MOLNÁR, Béla, 1182 Budapest (HU); FELEK, Roland, 2151 Fót (HU)
(74) Representative: Dwornik, Marek
(86) International application number: PCT/HU2023/050003
(87) International publication number: WO 2023/148514

(56) References cited:
- JP-B2- 4 095 248
- JP-B2- 4 871 737
- US-A- 4 814 279
- US-A1- 2003 059 349
- US-A1- 2003 190 744
- US-A1- 2010 031 757
- US-A1- 2020 188 904
- US-A1- 2020 319 067
- US-B2- 7 892 493
- US-B2- 8 173 068

## Description

The invention relates to a slide cover for covering a slide.

The invention further relates to an incubation chamber for staining a sample on a slide, comprising a slide cover according to the invention.

Microscopy of tissue samples on microscope slides is an important tool for pathological research. Digital pathology, which has become more widespread in recent decades, is a technique whereby glass slides are digitised using a scanner and the resulting digital images are analysed using computer programs.

As is well known, staining the sample is necessary to visualise certain tissue structures. Prior to and during staining, the sample on the slide is usually pretreated with reagent liquids according to a protocol in a series of successive steps, allowing specific detection of certain molecules. These methods include, inter alia, immunohistochemistry (IHC) or immunofluorescence (IF) techniques for the specific detection of various antigens based on antigen-antibody reactions and chromogenic or fluorescent in-situ hybridisation (CISH, FISH) methods for the specific detection of nucleic acids (e.g. DNA, RNA, mRNA, miRNA).

When using reagents, the prescribed incubation time and other parameters, including temperature and concentration, must be strictly observed. In addition, some of the reagents are harmful to health, so there are clear benefits to automating these workflows.

Since the reagents used for these sophisticated immuno- and hybridisation procedures are very expensive, it is preferable to use them in the smallest quantities possible. The challenge is therefore to spread the reagents efficiently on the biological sample and to ensure hermetic sealing to avoid sample drying.

International Publication WO 95110035, for example, describes an automatic staining machine in which the slides remain in the same position throughout the entire staining process and are successively manipulated by a moving manipulator head. The reagent is simply dripped onto the surface of the slide by the manipulator head, which spreads the reagent over the sample on its own. The disadvantage of this method is that it does not ensure uniform coverage of the entire sample and would require the use of a significant amount of reagent.

Various solutions are known in the state of the art for more efficient spreading of reagents on a sample. For example, the slide cover mentioned in US Patent No. US 4,985,206 and the slide cover device mentioned in US Patent No. US 2006/0051253 A1 and International Publication No. WO 2004/001389 A1 help to evenly distribute reagents over the sample and prevent evaporation by exploiting the capillary effect. When the latter solution is used in an automated apparatus, the reagents are dispensed by pipetting onto slides placed side by side in drawers, so that a pipette tip moving in X, Y, Z directions dispenses the reagent near the end of the slide cover, which is then soaked between the slide cover and the slide surface.

Other solutions are known e.g.: from document US 4 814 279 A disclosing a slide cover with a rigid frame.

In these solutions, the slide covers are of rigid design, i.e. the volume of the space defined by the surface of the slide and the slide cover is fixed. Since the slide cover must be suitable for different samples, and taking into account the different thicknesses of the samples, the height of the space is chosen so that the slide cover does not touch the sample even for thicker samples. Therefore, the volume defined by the state of the art slide covers is very large, i.e. 100-150 µl of reagent are usually used at a time to treat the samples. A further disadvantage of the current solutions is that liquid dispersion by capillary action is a slow process and requires the use of larger amounts of reagent to achieve uniform dispersion and avoid bubble formation. This obviously has a negative impact on the quality of the staining and the subsequent analysis of the sample.

The invention aims to provide a slide cover that is free from the disadvantages of prior art solutions. In particular, it is an object of the invention to provide a slide cover which requires the use of substantially less reagent than the present solutions and which allows the reagent to be spread more evenly over the sample.

The invention also aims to provide an incubation chamber comprising a slide cover according to the invention, whereby the staining steps can be automated and the parameters defined in the protocol (incubation time, incubation temperature, concentration, etc.) can be easily set and maintained for each reaction step with each reagent. In addition, the aim was to provide an incubation chamber that would allow the sample to be treated with several reagents in sequential order, under hermetic sealing.

We have recognised that the rigid design of the slide covers in currently known solutions only allows for uniform spreading of the reagent over the sample when larger amounts of reagent are used.

We have also recognised that by spreading the reagent on the sample using a flexible stretched membrane that is hermetically sealed around the sample, significantly less reagent (20-50 µl instead of 100-150 µl) is sufficient, and more uniform spreading can be achieved, concentrated locally on the sample. Minimal amounts of reagent are wasted in areas not covered by the sample, allowing better quality staining at lower cost.

The invention relates to a slide cover comprising a rigid frame, a flexible membrane stretched substantially flat by the rigid frame and fixed to the rigid frame, a lid for pressing the membrane against the slide, and a flexible element arranged between the lid and the rigid frame. The rigid frame is fixed to the lid by interposition of the flexible element to allow displacement relative to the lid, thereby ensuring self-adjustment of the membrane.

Furthermore, the invention relates to an incubation chamber comprising a block for supporting the slide and such a slide cover, the incubation chamber having an open position leaving the surface of the slide exposed and a closed position enclosing the slide between the block and the slide cover.

According to the invention, the object is achieved by a slide cover according to claim 1.

Furthermore, according to the invention, the object is achieved by an incubation chamber according to claim 12.

Some preferred embodiments of the invention are defined in the dependent claims. Further details of the invention will now be described with reference to the accompanying drawings. In the drawing is
Figure 1 is an exploded view of an exemplary slide cover according to the invention;
Figure 2a is a schematic perspective view of an exemplary embodiment of a slide cover according to the invention;
Figure 2b is a schematic view of the back of the slide cover according to Figure 2a;
Figure 3 is a view of the slide cover shown in Figure 2b without a lid;
Figure 4 is a schematic view of an exemplary embodiment of a rigid frame provided with a ridge stiffener according to the invention;
Figure 5a is a schematic cross-sectional view illustrating an exemplary membrane with an outer flange according to the invention;
Figure 5b is a schematic cross-sectional view illustrating the membrane according to Figure 5a pressed against the slide;
Figure 6 is a schematic cross-sectional view illustrating an exemplary membrane according to the invention with an inner flange in the state of the membrane being pressed against the slide;
Figure 7a is a schematic perspective view of an exemplary embodiment of an incubation chamber according to the invention in the open position of the chamber;
Figure 7b is a schematic perspective view of the incubation chamber in the open position of Figure 7a from another view, shown without the clamping lever;
Figure 8a is a schematic perspective view of the incubation chamber according to Figure 7a in the closed position of the chamber;
Fig. 8b is a schematic side view of the closed position incubation chamber according to Figure 8a, shown without the clamping lever.

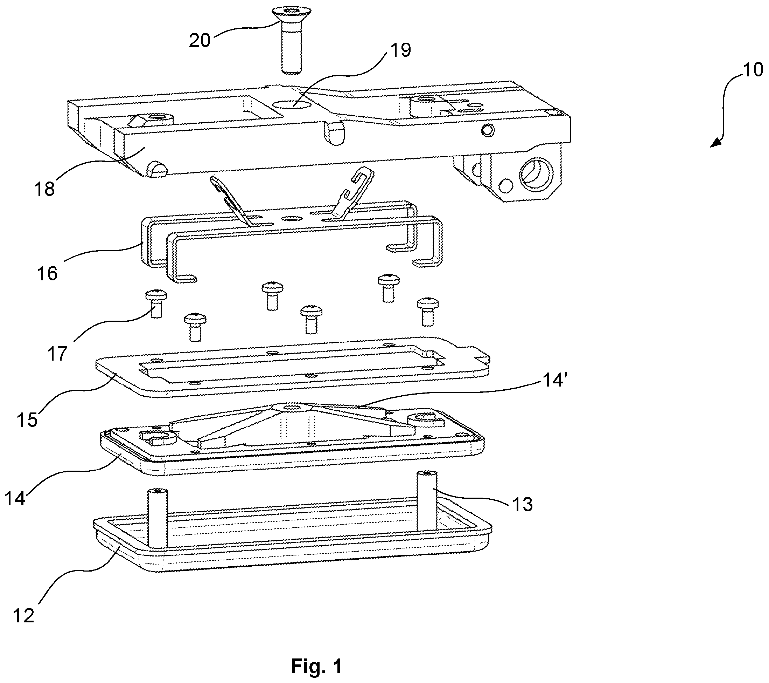

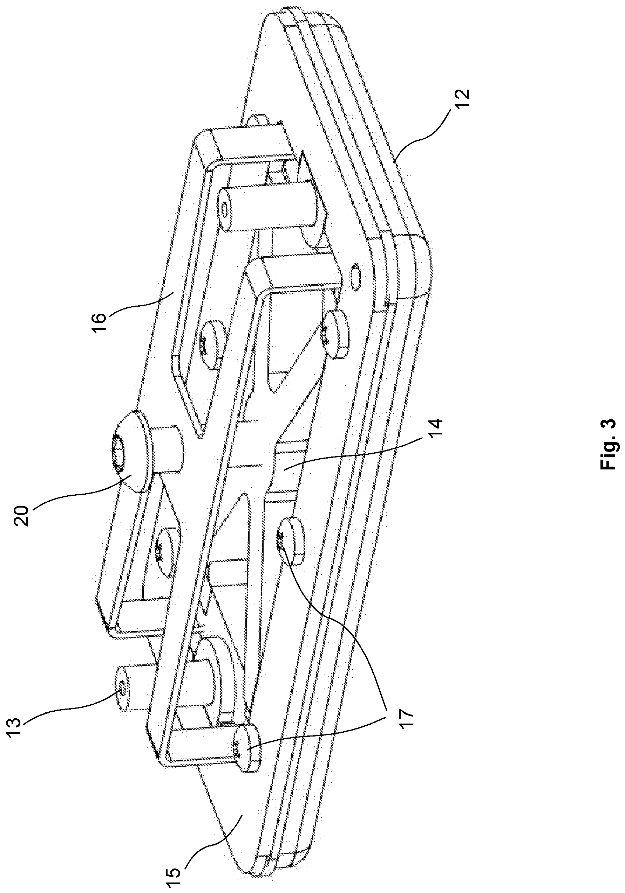

Figure 1 is an exploded view of an exemplary embodiment of a slide cover 10 according to the invention. The slide cover 10 is for uniformly spreading reagents on a microscope slide 30 and a sample thereon and hermetically sealing the prepared sample. The slide cover 10 comprises a rigid frame 14, a flexible membrane 12 stretched by the rigid frame 14 and fixed to the frame 14, a lid 18 for pressing the membrane 12 against the slide, and flexible element 16 arranged between the lid 18 and the frame 14. The membrane 12 for covering the slide 30 is preferably made of rubber, more preferably of hydrogenated nitrile butadiene rubber (HNBR), which is resistant to the corrosive action of various reagents while providing sufficient flexibility. It is noted that the membrane 12 may, of course, be made of other flexible materials such as PTFE, CTFE, FEP, FFKM or VITON, which are known flexible materials to the skilled person. The elastic membrane 12 is fixed to the frame 14 when stretched, i.e. the shape of the membrane 12 is essentially determined by the shape of the frame 14. Due to the stretching, the surface of the membrane 12 opposite to the frame 14 and facing the slide 30 during use of the device 10 is flat. In a particularly preferred embodiment shown in Figure 3, the membrane 12 is secured to the frame 14 by means of a fixing flange 15 such that the edge of the membrane 12 is sandwiched between the flange 15 and the frame 14, while the flange 15 is secured to the frame 14 by means of screws 17. This has the advantage that the membrane 12 can be easily replaced by unscrewing the screws 17 and removing the flange 15. In this embodiment, the flange 15 is formed as a thin metal plate. The membrane 12 may, of course, be attached to the frame 14 by other means, such as adhesive, but then replacement of the membrane 12 is more difficult.

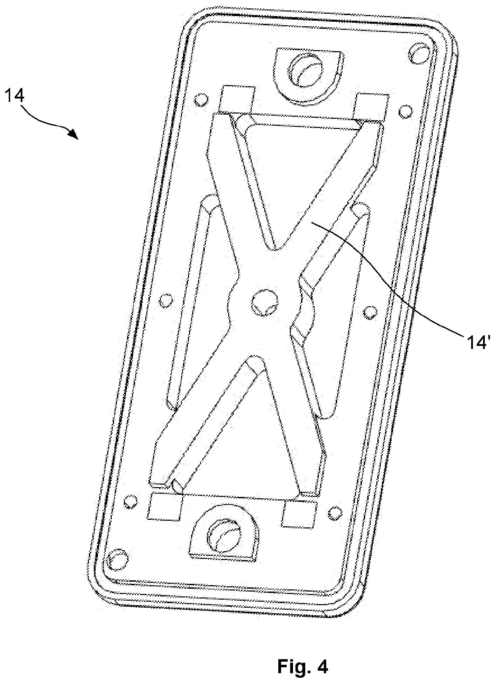

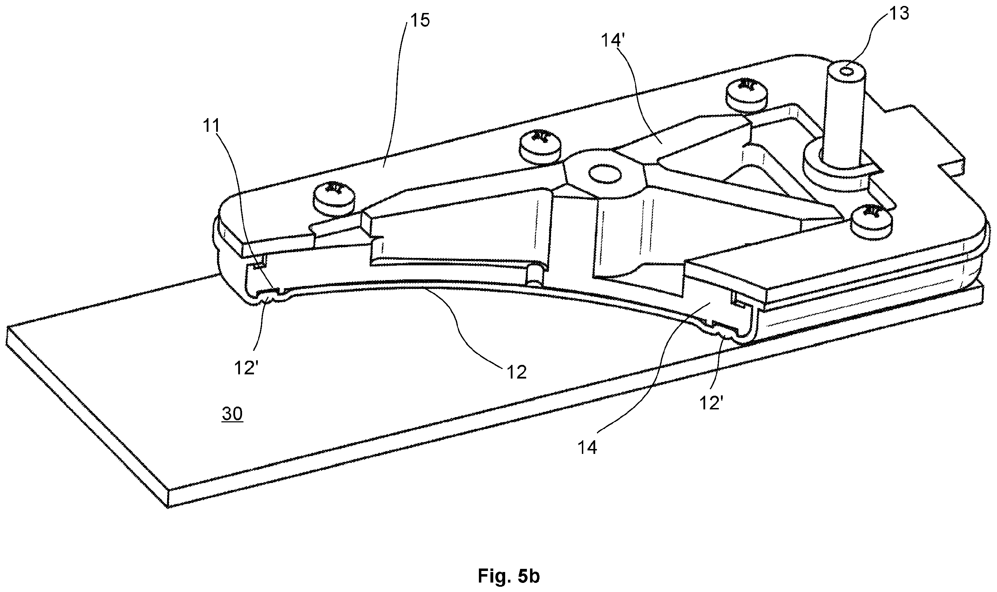

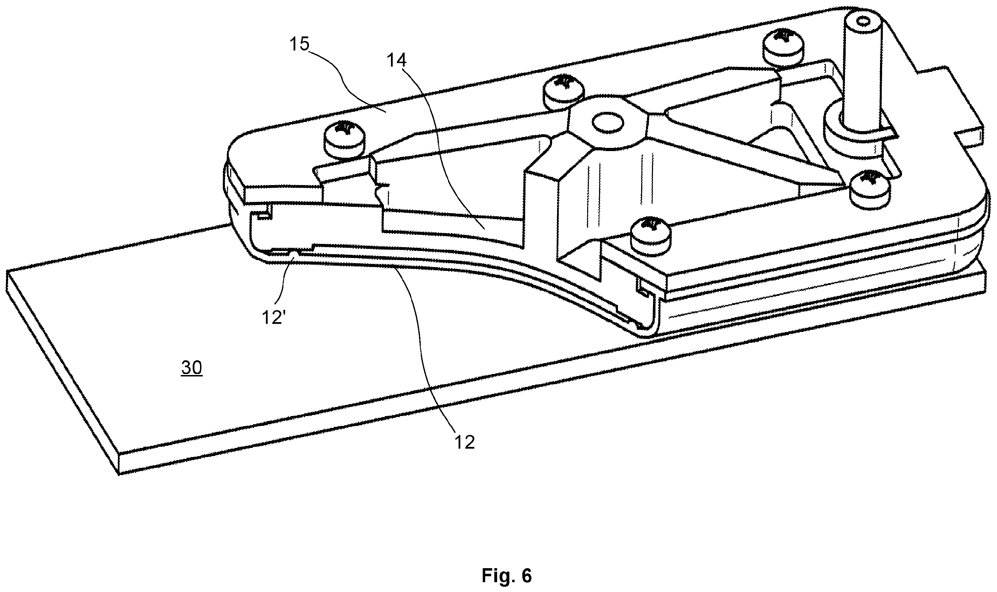

The stretched membrane 12 tends to deform the frame 14. By the rigidity of the frame 14, it is understood that the frame 14 holds its shape by resisting the forces exerted by the membrane 12. The frame 14 may preferably be made of, for example, engineering plastics such as polyoxymethylene (POM), polypropylene, poly(ether ether ketone), or metal such as aluminium. In order to increase the rigidity of the frame 14 and to reduce the amount of material used, the frame 14 is equipped with a ridge stiffener 14'. In the preferred embodiment shown in Figures 1 to 6, the frame 14 and the membrane 12 stretched therefrom correspond to the shape of the slide 30, being substantially rectangular in shape. The frame 14 and the membrane 12 are sized such that the stretched membrane 12 substantially completely covers the sample bearing surface of the slide 30, leaving only the gripping surface of the slide 30 exposed (see Figures 5b and 6). In the preferred embodiment shown in Figure 4, the frame 14 includes a cross-shaped ridge stiffener 14', wherein the stiffeners 14' connect the corners of the substantially rectangular frame 14. The stretched-out membrane 12 follows the shape of the rigid frame 14 such that the membrane 12 is stretched out by, and overlies, the edges of the frame 14. The frame 14 is configured to allow the central portion of the membrane 12 to deform towards the frame 14 under external force. In other words, the membrane 12 fixed to the frame 14 has a surface opposite the frame 14 which is flat in principle, but is capable of deforming elastically in the direction of the frame 14 under the action of an external force to assume a concave shape, as will be discussed below.

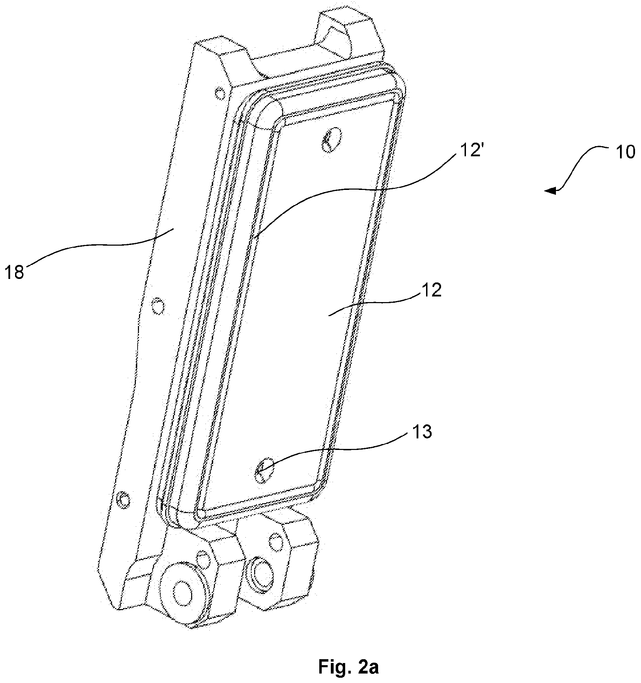

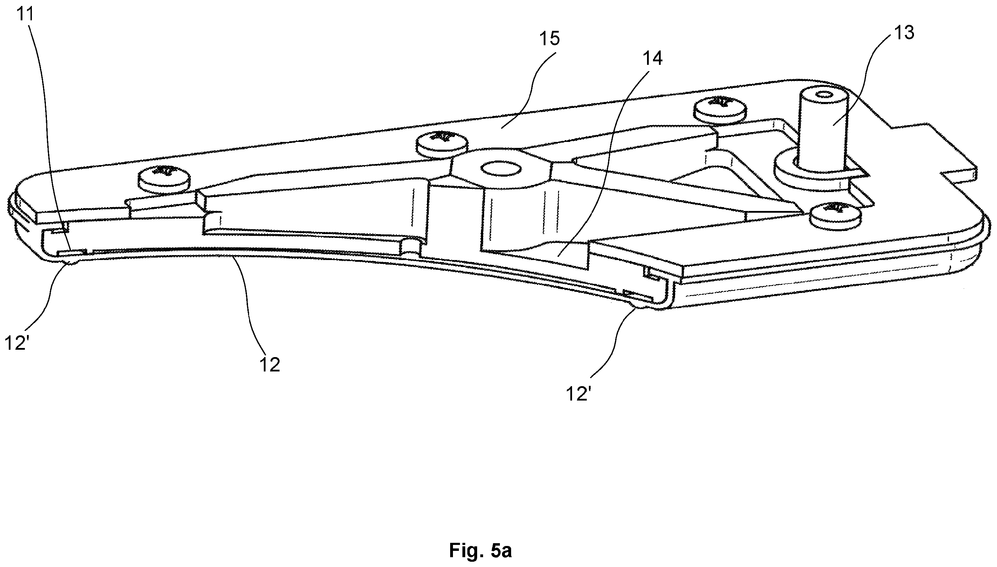

In a particularly preferred embodiment, the flexible membrane 12 is provided with a hermetically sealing flange 12' extending along the edge of the membrane 12. The flange 12' extends circumferentially along the entire circumference of the membrane 12, as can be observed, for example, in Figure 2a. In the context of the present description, hermetic sealing is understood to mean that, during use of the slide cover 10, when the membrane 12 is in contact with the surface of the slide 30, no liquid or gas passes between the flange 12' and the slide 30. For example, the flange 12' may be formed as an outer flange 12' shown in Figures 2a and 5a and 5b, i.e., here the flange 12' is an outer flange 12' formed on the surface of the membrane 12 opposite the rigid frame 14. Figure 5a illustrates an exemplary embodiment of an outer flange 12' in its default state, i.e., when the membrane 12 is not in contact with the slide 30. As can be seen, the flange 12' then protrudes from the surface of the membrane 12 opposite the frame 14. The state of the membrane 12 in contact with the slide 30 is illustrated in Figure 5b. In this embodiment, the frame 14 is preferably provided with a recess 11 extending circumferentially along the edge of the frame 14 to receive the outer flange 12', into which the flange 12' can be resiliently recessed under the compressive force exerted by the slide 30. The flange 12' and the surface of the slide 30 come into contact and a hermetic seal is formed between them. In the particularly preferred embodiment shown in Figure 6, the flange 12' is an inner flange 12' formed on the surface of the membrane 12 facing the frame 14 and protruding towards the frame 14. In this case, the frame 14 does not include the recess 11 and the flange 12' is not in direct contact with the surface of the slide 30. The hermetic seal is provided by the compressive force transmitted by the inner flange 12' between the membrane 12 and the slide 30. The advantage of this embodiment is that the surface of the membrane 12 opposite the frame 14 above the slide 30 is flat in its initial state, making it easier to clean compared to the outer flange 12' solution, and that fewer air bubbles remain under the membrane 12 when the membrane 12 is fitted to the slide 30, as will be discussed in more detail below.

In a particularly preferred embodiment, one or more, preferably two, fluidical connections 13 are formed on the flexible membrane 12, said one or more fluidical connections 13 extending through the membrane 12 and connecting a space at a side of the flexible membrane 12 facing the surface of the membrane 12 opposite the frame 14 to a space at a side of the membrane 12 facing the frame 14. Through the fluidical connection 13, a fluid, for example a reagent, may be introduced between the slide 30 and the membrane 12 attached thereto, and fluid may be removed therefrom without breaking the hermetic seal discussed above. In the preferred embodiment shown in Figure 2a, two fluidical connections 13 are formed on the membrane 12 at two spaced apart points on the membrane 12. In this way, the space between the membrane 12 and the slide 30 can be flushed substantially without breaking the hermetic seal by introducing the new reagent into the space through one of the fluidical connections 13 while removing the old reagent in the space through the other fluidical connection 13.

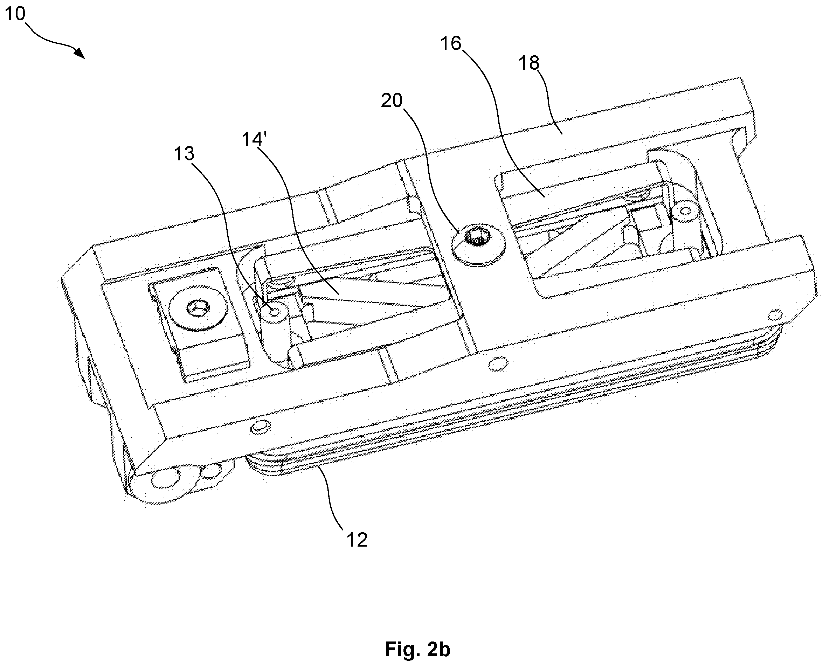

The lid 18 is used to press the membrane 12 against the slide 30, i.e. the compressive force between the membrane 12 and the slide 30 is provided by the lid 18. The lid 18 may be made of, for example, metal or other material of suitable strength, such as engineering plastic. In the slide cover 10 according to the invention, the rigid frame 14 is fixed to the lid 18 by allowing displacement relative to the lid 18 through the interposition of the flexible element 16. In the context of the present invention, the displacement of the frame 14 relative to the lid 18 is understood to be such that the plane of the membrane 12 can move relative to the lid 18 during contact with the slide 30, such that the frame 14 is parallel to the plane of the slide 30 at the moment of hermetic closure (see Figures 5b and 6). In this way, the plane defined by the flange 12' of the membrane 12 can be parallel to the plane of the slide 30 even if the position of the lid 18 would not allow this. In other words, the frame 14 and the membrane 12 are self-aligning under the compressive force exerted by the slide 30. As the frame 14 is fixed to the lid 18 by the interposition of the flexible element 16, the compressive force exerted by the lid 18 is distributed evenly along the edge of the frame 14, thus providing a more effective hermetic seal between the membrane 12 and the slide 30. In the particularly preferred embodiment shown in Figure 3, the flexible element 16 is a leaf spring, preferably a leaf spring fixed to the edge of the rigid frame 14, but of course other configurations of the flexible element 16 are also possible, e.g. rubber bellows, etc. For example, the flexible element 16, designed as a leaf spring, can be fixed to the frame 14 by means of the flange 15 mentioned above, while its other side rests on the lid 18 (see Figure 2b). The advantage of the design shown in Figure 3 is that the compressive force exerted by the lid 18 and transmitted by the flexible element 16 is transmitted to the four corners of the frame 14, thus achieving a very even distribution of forces. Note that the frame 14 is preferably attached to the lid 18 in such a way that the flexible element 16 is compressed (pre-tensioned) by a force of a few tenths to a few Newtons, i.e. tending to move the frame 14 away from the lid 18.

In a possible embodiment shown in Figures 1-3, the rigid frame 14 is secured to the lid 18 by means of a through hole 19 formed in the lid 18 and a hanging screw 20 threaded therethrough, such that the hanging screw 20 is screwed into the center of the cross-shaped ridge stiffener 14'. The displacement of the frame 14 relative to the lid 18 is ensured by the diameter of the through hole 19 being larger than the diameter of the hanging screw 20, but through which the head of the hanging screw 20 cannot pass. As a result, the plane of the membrane 12 can be tilted in any direction relative to the lid 18. It is noted that in this embodiment, the hanging screw 20 also passes through the flexible element 16 formed as a leaf spring, as is evident from Figure 1.

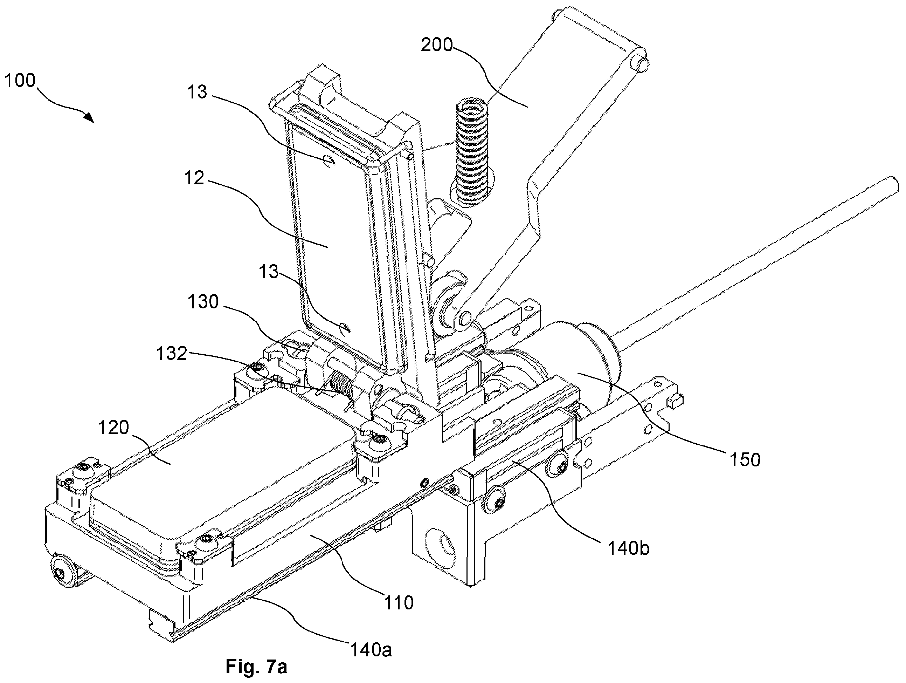

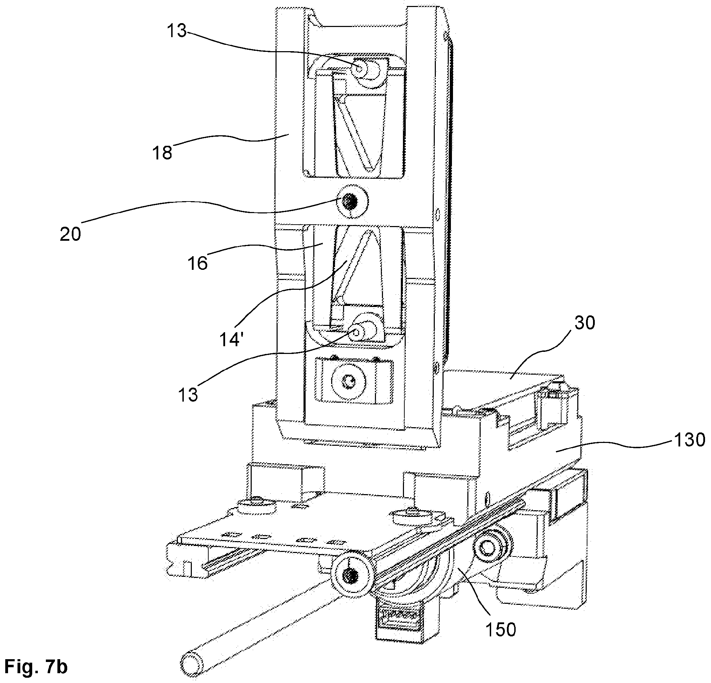

The invention also relates to an incubation chamber 100 for the treatment of sample on slide 30 (not shown) with reagents. The incubation chamber 100 comprises a block 110 for supporting the slide 30, and a slide cover 10 according to the invention. The block 110 may be made of, for example, metal or other material of suitable strength, such as engineering plastic. It is noted that in the context of the present description, the support of the slide 30 includes both direct and indirect support. In the former case, the slide 30 is in direct contact with the block 110 and in this case the surface of the block 110 in contact with the slide 30 is preferably flat, so that the slide 30 can rest stably on the block 110. However, in the preferred embodiments shown in Figures 7a-8b, indirect support is provided in such a way that the block 110 is provided with a heating unit 120 for heating the slide 30, i.e. the slide 30 is in contact with the heating unit 120 and rests thereon. The surface of the heating unit 120 in contact with the slide 30 is preferably flat for stable support and to maximise heat transfer, as can be observed in Figure 7a. The heating unit 120 may be, for example, a filament heater or a Peltier heater, as is known to the skilled person. It is noted that embodiments comprising a heating unit 120 may be envisaged (not shown in the figures), wherein the slide 30 is in direct contact with the block 110 and the heating body 120 heats the slide 30 via the block 110. In this case, of course, the block 110 should preferably be made of a material with good thermal conductivity properties, such as metal, as is obvious to the person skilled in the art.

In the incubation chamber 100 according to the invention, the slide cover 10 is arranged to be displaceable relative to said block 110 such that the incubation chamber 100 has an open position leaving the surface of the slide 30 opposite the block 110 exposed and a closed position enclosing the slide 30 between the block 110 and the slide cover 10. In the context of the present description, the surface of the slide 30 opposite the block 110 is understood to be the upwardly facing sample containing surface of the slide 30 supported by the block 110 and facing the membrane 12 in the closed position. The open position of the incubation chamber 100 is illustrated in Figures 7a-7b, in which the sample is freely accessible and can be manipulated, for example by dropping reagent onto it.

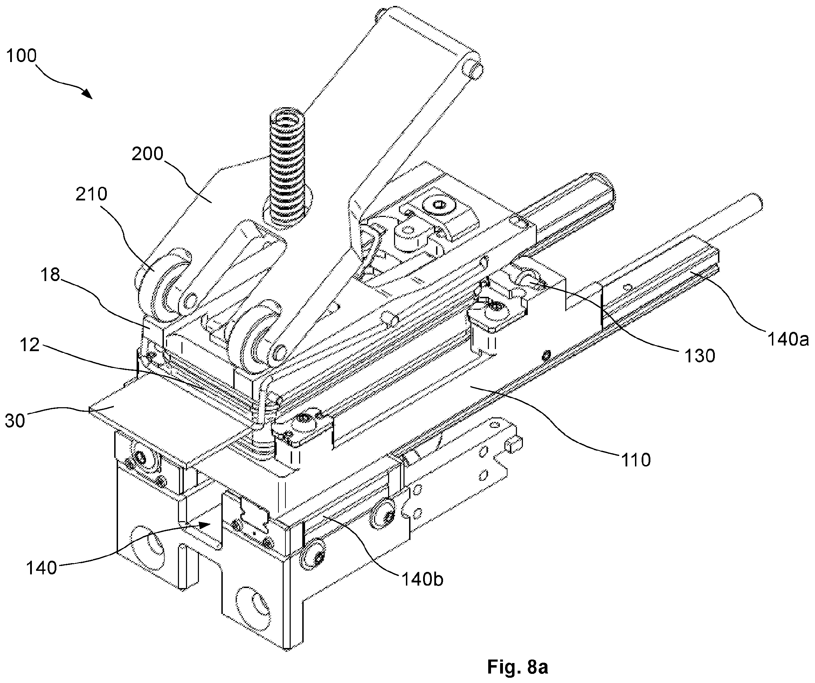

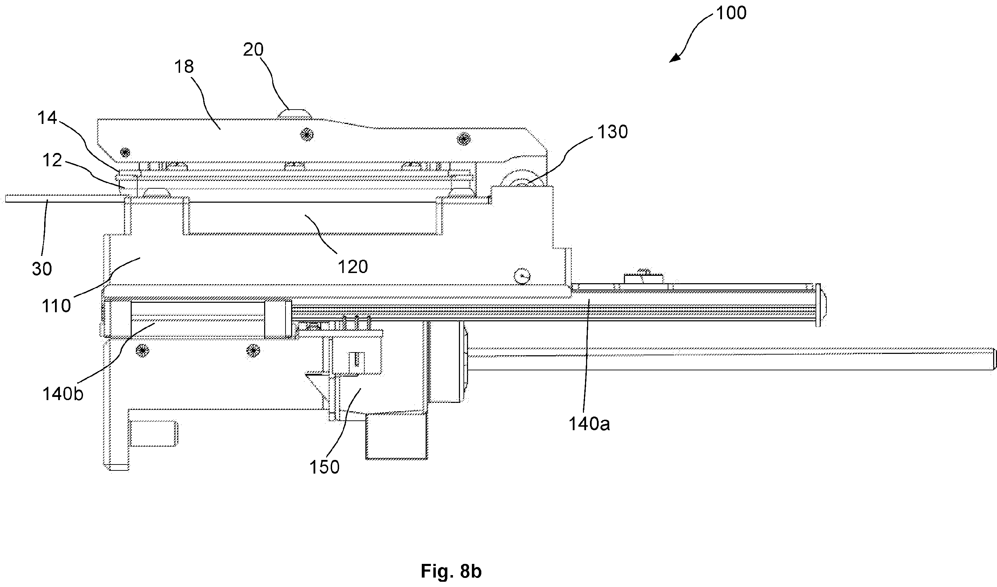

The closed position of the incubation chamber 100 is shown in Figures 8a-8b, in which closed position, the surface of the membrane 12 opposite the frame 14 and the surface of the slide 30 opposite the block 110 together define a closed incubation space enclosing the sample and reagent. The shape and volume of the incubation space can adapt to the height of the sample on the respective slide 30 and the height of the liquid film above the sample due to the flexibility of the membrane 12. The height of the space is typically a few tenths of a mm. Figure 8b clearly shows that in the closed position, the plane of the rigid frame 14 is substantially parallel to the plane of the slide 30, creating a hermetic seal between the flange 12' of the membrane 12 and the slide 30. By the closed nature of the incubation space, it is understood that the flow of gas or liquid between the space and the outside is possible at most only through fluidical connections 13. A pump (not shown in the figures) can be connected to the fluidical connections 13 via tubes to control the flow of fluid through the fluidical connections 13 and to ensure the sealing of the incubation space.

In the preferred embodiment shown in Figures 7a-8b, the slide cover 10 is attached to the block 110 by means of a hinge 130, i.e., the slide cover 10 can pivot about the axis of the hinge 130 relative to the block 110 between open and closed positions as end positions. Preferably, the hinge 130 is provided with a spring 132 which tends to urge the slide cover 10 towards the open position, as shown, for example, in Figure 7a. The block 110 is preferably configured to slide along a track system 140. The track system 140 comprises a moving member 140a and a stationary member 140b fixed to the block 110. It is noted that although in the embodiment shown in Figures 7a-8b, the block 110 is connected to two linear track systems 140, an embodiment comprising a single or more than two track systems 140 is also conceivable. The stationary member 140b of the track system 140 is, by analogy, attachable to an external device such as a microscope or a staining machine (not shown), so that the block 110, together with the slide cover 10 attached thereto, is movable relative to the stationary member 140b attached to the external device.

In a particularly preferred embodiment, the incubation chamber 100 includes a clamping lever 200 for forcing the slide cover 10 from an open position to a closed position by rotating the slide cover 10 about the hinge 130 during displacement of the block 110. The clamping lever 200 is configured such that its position relative to the stationary member 140b is fixed. The clamping lever 200 may also preferably be secured to an external device in a similar manner to the stationary member 140b. As shown in Figure 7a, in one end state of the track system 140 corresponding to the open position of the incubation chamber 100, the clamping lever 200 is not in contact with the slide cover 10 and does not exert a compressive force thereon. As the block 110 is moved along the track system 140 towards the clamping lever 200, the block 110 gradually slides under the clamping lever 200, causing the slide cover 10 to pivot about the hinge 130 towards the closed position. The clamping lever 200 may preferably be provided with rollers 210 which, by rolling along the lid 18 of the slide cover 10, facilitate pivoting of the slide cover 10 about the hinge 130. The tilting of the slide cover 10 is prevented by the spring 132 which tends to resist, but cannot overcome, the force exerted by the clamping lever 200. In the other end state of the track system 140 illustrated in Figure 8a, the incubation chamber 100 is closed and the closed incubation space between the slide 30 and the membrane 12 is created. Displacement of the block 110 along the track system 140 is preferably provided by means of a stepper motor 150.

Using the exemplary embodiment of incubation chamber 100, the slide 30 containing the sample to be stained is placed directly on the block 110 in the open state of incubation chamber 100 or on the heating unit 120 depending on the embodiment. The slide cover 10 is held in the open position by the spring 132. In this state, a small amount of reagent (typically 20-50 µl) can be pipetted onto the surface of the slide 30. The block 110 is then moved along the track system 140 by means of the stepper motor 150 in such a way that the clamping lever 200 rotates the slide cover 10 around the hinge 130 while passing along the lid 18 of the slide cover 10. As the block 110 is moved, the angle between the membrane 12 and the surface of the slide 30 decreases until it is zero in the closed position. As the angle between the membrane 12 and the slide 30 decreases, the membrane 12 spreads the reagent liquid placed on the slide 30 evenly due to its elasticity, and displaces the air between the membrane 12 and the slide 30. The flexible membrane 12 exerts a hydraulic force on the liquid column, which causes the reagent liquid to spread evenly over the sample. In other words, the membrane 12 is pressed against the slide 30, causing the reagent to fill the area under the membrane 12, the incubation space. In this way, a uniform, bubble-free staining can be achieved with a minimum amount of reagent. The pressure force created by the clamping lever 200 and transmitted by the lid 18 is uniformly transmitted to the membrane 12 due to the inventive construction of the slide cover 10, so that a perfect fit and hermetic seal between the membrane 12 and the slide 30 can be achieved in the closed position of the incubation chamber 100.

In the closed position, the heating unit 120 can be used to set the desired temperature for incubation and to introduce buffer fluids or additional reagents for the staining process into the incubation space via the fluidical connections 13. At the end of the staining process, by moving the block 110 in the opposite direction along the track system 140, the slide cover 10 can be slid out from under the clamping lever 200. The spring 132 moves the slide cover 10 to the open position so that the slide 30 can be removed from the incubation chamber 100.

It will be apparent to those skilled in the art that alternative solutions to the other embodiments shown herein are contemplated, but fall within the scope of protection defined by the claims.

## Claims

1. A slide cover (10), comprising
- a rigid frame (14), and **characterized by** further comprising:
- a flexible membrane (12) stretched by and fixed to the rigid frame (14),
- a lid (18) for pressing the membrane (12) against a slide (30), and
- a flexible element (16) arranged between the lid (18) and the rigid frame (14),
which rigid frame (14) is attached to the lid (18) by means of the flexible element (16) in a such a way that allows displacement relative to the lid (18).

2. The slide cover (10) according to claim 1, **characterized in that** the rigid frame (14) and the membrane (12) stretched thereby correspond to the shape of the slide (30) and are substantially rectangular.

3. The slide cover (10) according to claim 1 or 2, **characterized in that** one or more fluidical connections (13) are formed on the flexible membrane (12), said one or more fluidical connections (13) extending through the membrane (12) and connecting a space at a side of the flexible membrane (12) facing the surface of the membrane (12) opposite the frame (14) to a space at a side of the membrane (12) facing the frame (14).

4. The slide cover (10) according to any one of claims 1 to 3, **characterized in that** the flexible membrane (12) is fixed to the rigid frame (14) by means of a fixing flange (15).

5. The slide cover (10) according to any one of claims 1 to 4, **characterized in that** the rigid frame (14) is attached to the lid (18) by means of a through hole (19) formed in the lid (18) and a hanging screw (20) passed therethrough, the diameter of the through hole (19) being larger than the diameter of the hanging screw (20).

6. The slide cover (10) according to any one of claims 1 to 5, **characterized in that** the rigid frame (14) is provided with a ridge stiffener (14'), preferably a cross-shaped ridge stiffener (14').

7. The slide cover (10) according to any one of claims 1 to 6, **characterized in that** the flexible element (16) is a leaf spring, preferably a leaf spring fixed to the edges of the rigid frame (14).

8. The slide cover (10) according to any one of claims 1 to 7, **characterized in that** the flexible membrane (12) is provided with a hermetically sealing flange (12') extending along the edge of the membrane (12).

9. The slide cover (10) according to claim 8, **characterized in that** the flange (12') is an inner flange (12') formed on the surface of the membrane (12) facing the rigid frame (14) and protruding towards the frame (14).

10. The slide cover (10) according to claim 8, **characterized in that** the flange (12') is an outer flange (12') formed on a surface of the membrane (12) opposite the rigid frame (14), and the frame (14) is preferably provided with a recess (11) for receiving the outer flange (12').

11. The slide cover (10) according to any one of claims 1 to 10, **characterized in that** the flexible membrane (12) is made of rubber, preferably hydrogenated nitrile butadiene rubber.

12. An incubation chamber (100) for treating a sample on a slide (30) with a reagent, **characterized in that** it comprises a block (110) for supporting the slide (30) and a slide cover (10) according to any one of claims 1 to 11, said slide cover (10) being arranged to be displaceable relative to said block (110) such that the incubation chamber (100) has an open position leaving the surface of the slide (30) opposite the block (110) exposed and a closed position enclosing the slide (30) between the block (110) and the slide cover (10), wherein in the closed position the surface of the membrane (12) opposite the frame (14) and the surface of the slide (30) opposite the block (110) together define a closed incubation space.

13. The incubation chamber (100) according to claim 12, **characterized in that** the block (110) is provided with a heating unit (120) for heating the slide (30).

14. The incubation chamber (100) according to claim 12 or 13, **characterized in that** the slide cover (10) is attached to the block (110) by means of a hinge (130).

15. The incubation chamber (100) according to claim 14, **characterized in that** the block (110) is configured to be slidable along a track system (140), and the incubation chamber (100) comprises a clamping lever (200) for forcing the slide cover (10) from an open position to a closed position by rotating the slide cover (10) about the hinge (130) during sliding of the block (110).

## Patentansprüche

1. Schiebeabdeckung (10), umfassend
- einen starren Rahmen (14), und **dadurch gekennzeichnet, dass** sie ferner umfasst:
- eine flexible Membran (12), die durch den starren Rahmen (14) gespannt und daran befestigt ist,
- einen Deckel (18) zum Drücken der Membran (12) gegen einen Schieber (30), und
- ein flexibles Element (16), das zwischen dem Deckel (18) und dem starren Rahmen (14) angeordnet ist,
wobei der starre Rahmen (14) mittels des flexiblen Elements (16) derart an dem Deckel (18) befestigt ist, dass eine Verschiebung relativ zu dem Deckel (18) ermöglicht wird.

2. Schiebeabdeckung (10) nach Anspruch 1, **dadurch gekennzeichnet, dass** der starre Rahmen (14) und die dadurch gespannte Membran (12) der Form des Schiebers (30) entsprechen und im Wesentlichen rechteckig sind.

3. Schiebeabdeckung (10) nach Anspruch 1 oder 2, **dadurch gekennzeichnet, dass** eine oder mehrere Fluidverbindungen (13) an der flexiblen Membran (12) ausgebildet sind, wobei sich die eine oder die mehreren Fluidverbindungen (13) durch die Membran (12) erstrecken und einen Raum an einer Seite der flexiblen Membran (12), die der Oberfläche der Membran (12) gegenüber dem Rahmen (14) zugewandt ist, mit einem Raum an einer Seite der Membran (12), die dem Rahmen (14) zugewandt ist, verbinden.

4. Schiebeabdeckung (10) nach einem der Ansprüche 1 bis 3, **dadurch gekennzeichnet, dass** die flexible Membran (12) mittels eines Befestigungsflansches (15) an dem starren Rahmen (14) befestigt ist.

5. Schiebeabdeckung (10) nach einem der Ansprüche 1 bis 4, **dadurch gekennzeichnet, dass** der starre Rahmen (14) mittels eines Durchgangslochs (19), das in dem Deckel (18) ausgebildet ist, und einer dadurch geführten Hängeschraube (20) an dem Deckel (18) befestigt ist, wobei der Durchmesser des Durchgangslochs (19) größer als der Durchmesser der Hängeschraube (20) ist.

6. Schiebeabdeckung (10) nach einem der Ansprüche 1 bis 5, **dadurch gekennzeichnet, dass** der starre Rahmen (14) mit einer Firstversteifung (14'), vorzugsweise einer kreuzförmigen Firstversteifung (14'), versehen ist.

7. Schiebeabdeckung (10) nach einem der Ansprüche 1 bis 6, **dadurch gekennzeichnet, dass** das flexible Element (16) eine Blattfeder ist, vorzugsweise eine Blattfeder, die an den Rändern des starren Rahmens (14) befestigt ist.

8. Schiebeabdeckung (10) nach einem der Ansprüche 1 bis 7, **dadurch gekennzeichnet, dass** die flexible Membran (12) mit einem hermetisch abdichtenden Flansch (12') versehen ist, der sich entlang des Rands der Membran (12) erstreckt.

9. Schiebeabdeckung (10) nach Anspruch 8, **dadurch gekennzeichnet, dass** der Flansch (12') ein innerer Flansch (12') ist, der an der Oberfläche der Membran (12) ausgebildet ist, die dem starren Rahmen (14) zugewandt ist und in Richtung des Rahmens (14) vorsteht.

10. Schiebeabdeckung (10) nach Anspruch 8, **dadurch gekennzeichnet, dass** der Flansch (12') ein äußerer Flansch (12') ist, der an einer Oberfläche der Membran (12) gegenüber dem starren Rahmen (14) ausgebildet ist, und der Rahmen (14) vorzugsweise mit einer Aussparung (11) zum Aufnehmen des äußeren Flansches (12') versehen ist.

11. Schiebeabdeckung (10) nach einem der Ansprüche 1 bis 10, **dadurch gekennzeichnet, dass** die flexible Membran (12) aus Kautschuk, vorzugsweise hydriertem Nitrilbutadienkautschuk, hergestellt ist.

12. Inkubationskammer (100) zum Behandeln einer Probe auf einem Schieber (30) mit einem Reagenz, **dadurch gekennzeichnet, dass** sie einen Block (110) zum Tragen des Schiebers (30) und eine Schiebeabdeckung (10) nach einem der Ansprüche 1 bis 11 umfasst, wobei die Schiebeabdeckung (10) so angeordnet ist, dass sie relativ zu dem Block (110) verschiebbar ist, so dass die Inkubationskammer (100) eine offene Position, in der die Oberfläche des Schiebers (30) gegenüber dem Block (110) freiliegt, und eine geschlossene Position, in der der Schieber (30) zwischen dem Block (110) und der Schiebeabdeckung (10) eingeschlossen ist, aufweist, wobei in der geschlossenen Position die Oberfläche der Membran (12) gegenüber dem Rahmen (14) und die Oberfläche des Schiebers (30) gegenüber dem Block (110) zusammen einen geschlossenen Inkubationsraum definieren.

13. Inkubationskammer (100) nach Anspruch 12, **dadurch gekennzeichnet, dass** der Block (110) mit einer Heizeinheit (120) zum Erwärmen des Schiebers (30) versehen ist.

14. Inkubationskammer (100) nach Anspruch 12 oder 13, **dadurch gekennzeichnet, dass** die Schiebeabdeckung (10) mittels eines Scharniers (130) an dem Block (110) befestigt ist.

15. Inkubationskammer (100) nach Anspruch 14, **dadurch gekennzeichnet, dass** der Block (110) so konfiguriert ist, dass er entlang eines Schienensystems (140) verschiebbar ist, und die Inkubationskammer (100) einen Klemmhebel (200) zum Zwingen der Schiebeabdeckung (10) aus einer offenen Position in eine geschlossene Position durch Drehen der Schiebeabdeckung (10) um das Scharnier (130) während des Verschiebens des Blocks (110) umfasst.

## Revendications

1. Couvercle de lame (10) comprenant:
- un cadre rigide (14), et **caractérisé en ce qu'**il comprend en outre:
- une membrane souple (12) étirée par le cadre rigide (14) et fixée sur celui-ci,
- un capot (18) pour presser la membrane (12) contre une lame (30), et
- un élément flexible (16) placé entre le capot (18) et le cadre rigide (14),
lequel cadre rigide (14) est attaché au capot (18) au moyen de l'élément flexible (16) d'une manière qui permet le déplacement par rapport au capot (18).

2. Couvercle de lame (10) selon la revendication 1, **caractérisé en ce que** le cadre rigide (14) et la membrane (12) étirée par ce dernier correspondent à la forme de la lame (30) et sont substantiellement rectangulaires.

3. Couvercle de lame (10) selon la revendication 1 ou 2, **caractérisé en ce que** une ou plusieurs connexions fluidiques (13) sont formées sur la membrane souple (12), lesdites une ou plusieurs connexions fluidiques (13) s'étendant à travers la membrane (12) et reliant un espace situé sur un côté de la membrane flexible (12) faisant face à la surface de la membrane (12) à l'opposé du cadre (14) à un espace situé sur un côté de la membrane (12) faisant face au cadre (14).

4. Couvercle de lame (10) selon l'une quelconque des revendications 1 à 3, **caractérisé en ce que** la membrane souple (12) est fixée au cadre rigide (14) au moyen d'une flasque de fixation (15).

5. Couvercle de lame (10) selon l'une quelconque des revendications 1 à 4, **caractérisé en ce que** le cadre rigide (14) est attaché au capot (18) au moyen d'un trou débouchant (19) formé dans le capot (18) et d'une vis suspendue (20) passant dans ce dernier, le diamètre du trou débouchant (19) étant plus grand que le diamètre de la vis suspendue (20).

6. Couvercle de lame (10) selon l'une quelconque des revendications 1 à 5, **caractérisé en ce que** le cadre rigide (14) est pourvu d'un raidisseur à arêtes (14'), de préférence un raidisseur à arêtes en croix (14').

7. Couvercle de lame (10) selon l'une quelconque des revendications 1 à 6, **caractérisé en ce que** l'élément flexible (16) est un ressort à lame, de préférence un ressort à lame fixé sur les bords du cadre rigide (14).

8. Couvercle de lame (10) selon l'une quelconque des revendications 1 à 7, **caractérisé en ce que** la membrane souple (12) est pourvue d'une bride d'étanchéité (12') qui s'étend le long du bord de la membrane (12).

9. Couvercle de lame (10) selon la revendication 8, **caractérisé en ce que** la bride (12') est une bride intérieure (12') formée sur la surface de la membrane (12) qui fait face au cadre rigide (14) et faisant saillie vers le cadre (14).

10. Couvercle de lame (10) selon la revendication 8, **caractérisé en ce que** la bride (12') est une bride extérieure (12') formée sur une surface de la membrane (12) située à l'opposé du cadre rigide (14), et le cadre (14) est de préférence pourvu d'un évidement (11) destiné à recevoir la bride extérieure (12').

11. Couvercle de lame (10) selon l'une quelconque des revendications 1 à 10, **caractérisé en ce que** la membrane souple (12) est en caoutchouc, de préférence du caoutchouc butadiène-nitrile hydrogéné.

12. Chambre d'incubation (100) pour traiter un échantillon sur une lame (30) avec un réactif, **caractérisée en ce qu'**elle comprend un bloc (110) destiné à supporter la lame (30) et un couvercle de lame (10) selon l'une quelconque des revendications 1 à 11, ledit couvercle de lame (10) étant agencé pour pouvoir être déplacé par rapport audit bloc (110) de telle manière que la chambre d'incubation (100) a une position ouverte laissant la surface de la lame (30) à l'opposé du bloc (110) exposée et une position fermée enfermant la lame (30) entre le bloc (110) et le couvercle de lame (10), dans laquelle, dans la position fermée, la surface de la membrane (12) à l'opposé du cadre (14) et la surface de la lame (30) à l'opposé du bloc (110) définissent ensemble un espace d'incubation fermé.

13. Chambre d'incubation (100) selon la revendication 12, **caractérisée en ce que** le bloc (110) est pourvu d'une unité de chauffage (120) pour chauffer la lame (30).

14. Chambre d'incubation (100) selon la revendication 12 ou 13, **caractérisée en ce que** le couvercle de lame (10) est attaché au bloc (110) au moyen d'une charnière (130).

15. Chambre d'incubation (100) selon la revendication 14, **caractérisée en ce que** le bloc (110) est configuré pour pouvoir coulisser le long d'un système de rail (140), et la chambre d'incubation (100) comprend un levier de serrage (200) pour contraindre le couvercle de lame (10) d'une position ouverte vers une position fermée en faisant tourner le couvercle de lame (10) autour de la charnière (130) pendant le coulissement du bloc (110).
